# EUROPÄISCHE PATENTSCHRIFT

(11) **EP 1 792 980 B1**
(45) Veröffentlichungstag und Bekanntmachung des Hinweises auf die Patenterteilung: **01.12.2010**
(21) Anmeldenummer: 07002971.5
(22) Anmeldetag: 29.05.2001
(51) Int. Cl.: G01N 33/50

(54) **Kollagenbiomatrix und Verfahren zu ihrer Herstellung**
Collagen biomatrix and methods to poduce it
Matrice de collagène et procédure de la production

(30) Priorität: 31.05.2000 DE 10026789
(43) Veröffentlichungstag der Anmeldung: 06.06.2007
(62) Teilanmeldung aus: 01953164.9
(73) Patentinhaber: Arthro Kinetics AG, 73728 Esslingen (DE)
(72) Erfinder: Noll, Michaela, 70597 Stuttgart (DE); Schandar, Markus, 71720 Oberstenfeld (DE); Graeve, Thomas, 70372 Stuttgart (DE)
(74) Vertreter: Schwahn, Hartmut

(56) Entgegenhaltungen:
- EP-A- 0 851 227
- EP-A1- 1 027 897
- WO-A-91/16010
- WO-A-97/41208
- WO-A-98/51317
- US-A- 5 714 582
- US-A- 5 945 101

## Beschreibung

Die Erfindung betrifft eine Biomatrix sowie Verfahren zu ihrer Herstellung.

Gelenkerkrankungen stellen eine weitverbreitete und mit einer Vielzahl von Beschwernissen für die betroffenen Personen verbundene Krankheit dar. So spielen Knochen- und Knorpeldefekte eine zentrale Rolle bei der Pathogenese von Arthrose, aber auch bei posttraumatischen Zuständen und ausgelockerten Endoprothesen. Derartige Defekte können durch Einsatz autologer oder homologer Knochen beziehungsweise Knorpel beziehungsweise entsprechender Ersatzmaterialien therapiert werden. Während autologe Materialien nicht unbegrenzt zur Verfügung stehen, müssen bei homologen Transplantaten infektiologische Gesichtspunkte berücksichtigt werden.

Das Hauptproblem degenerativer oder traumatischer Gelenkerkrankungen besteht darin, dass der geschädigte Gelenkknorpel nur eine geringe Fähigkeit zu Regeneration zeigt. Es ist bekannt, derartige Knorpeldefekte mittels der autologen Chondrocytentransplantation (ACT) als biologischem Therapieverfahren zu behandeln. Mit diesem Therapieverfahren konnte erstmals die Neubildung von hyalinem Knorpel in einem Gelenkknorpeldefekt nachgewiesen werden.

Das Prinzip dieser Methode basiert darauf, in vitro kultivierte Knorpelzellen des Patienten nach Debridement des degenerierten Knorpels unter einen auf den Defekt genähten Periostlappen zu spritzen (Peterson-Methode). Das Verfahren ist technisch anspruchsvoll und wirft methodische Probleme auf; so ist beispielsweise der längerfristige Verbleib der Knorpelzellen im Defekt nicht garantiert. Des Weiteren ist unklar, ob die kultivierten Knorpelzellen zum Zeitpunkt der Transplantation in der Lage sind, die für die dauerhafte Defektfüllung benötigte Matrix zu bilden. Die transplantierten Zellen befinden sich zumeist in einem dedifferenzierten Zustand und weisen morphologische und physiologische Gemeinsamkeiten mit Fibroblasten auf.

Aus der DE 197 21 661 A1 ist es bekannt, neuartige Materialien für den Ersatz von Knochen oder Knorpeln, die sich durch eine spezifische Struktur an sich bekannter Werkstoffe auszeichnen, zu verwenden. Es handelt sich um ein Knochen- beziehungsweise Knorpelimplantat auf Basis eines dreidimensionalen Gitters, das im Wesentlichen aus einer Vielzahl von regelmäßig angeordneten Stäben aus einem teilweise oder vollständig bioresorbierbaren Werkstoff besteht. Die Stäbe bilden eine geometrische dreidimensionale Struktur aus, wobei diese Struktur in Bezug auf Elastizität und Festigkeit auf das Gewebe abgestimmt ist, das es im Körper des Patienten ersetzen soll. Die Stäbe können aus Poly-D-Lactiden, Poly-L-Lactiden, Poly-DL-Lactiden, Hydroxylapatiten, Calciumphosphaten oder Mischungen dieser Stoffe bestehen, die im Wesentlichen Calciumphosphate beziehungsweise Hydroxylapatite, Kollagen, Agar oder Gelatine enthalten.

Die WO 99/08728 offenbart ein osteoinduktives oder chondroinduktives Faktorengemisch, das eingebettet in Nanosphären vorliegt. Offenbart wird auch ein System umfassend eine bioabbaubare Matrix, enthaltend z.B. Kollagen Typ I oder Typ II und darin eingebettet die von den Nanosphären umhüllten Faktoren. Die Nanosphären sind als Polymerpartikel ausgeführt.

Aus der WO 95/33821 ist es bekannt, dreidimensionale Strukturen aus Kollagen herzustellen, wobei die interstitiellen Bereiche durch z.B. Fibroblasten oder Chondrocyten überbrückt werden und eine Kultivierung dieses Netzwerkes in einem Kulturmedium durchgeführt wird. In dieser Druckschrift wird die Kultivierung der genannten Zellen auf diesem dreidimensionalen Netzwerk beschrieben ebenso wie die Verwendung dieses künstlichen Gewebes als Knorpelersatz.

Die WO 98/17791 beschreibt die Isolierung und die Verwendung von Vorläufer-Chondrocyten, die in Kultur methodisch vermehrt und zur Verwendung therapeutisch einsetzbaren Knorpelgewebes verwendet werden können. Auch hier wird die Kultivierung von Chondrocyten auf einem dreidimensionalen Netzwerk beschrieben, wobei die interstitiellen Räume durch die Chondrocyten überbrückt werden.

Die WO 99/00152 offenbart ein Verfahren zur Herstellung eines bioartifiziellen Transplantates, wobei aus einem allogenen oder xenogenen Gewebe alle antigen-reaktiven Zellen durch enzymatische oder chemische Behandlung entfernt werden und das so gewonnene zellfreie, nicht-denaturierte Material mit gewünschten autologen Zellen besiedelt wird, wodurch ein unmittelbar einsatzbereites Transplantat erhalten wird.

Bei den bekannten Verfahren wirkt sich nachteilig aus, dass transplantierte oder kultivierte Knorpelzellen ihre ursprüngliche Syntheseleistung nicht wieder erreichen.

Die in der Interzellularsubstanz des Knorpels eingelagerten Knorpelzellen -die Chondrocyten- dedifferenzieren nämlich unter in vitro Kulturbedingungen im Laufe der Kultivierungsdauer. Während primäre Knorpelzellen (P0 Kultur) nach ihrer Isolation aus Knorpelgewebe noch knorpelzelltypische Syntheseleitungen, wie die Bildung von Kollagen Typ II, Typ IX und Typ XI, zeigen, verlieren kultivierte Zellen diese typischen Eigenschaften während weiterer Passagen (P1-PX) in vitro.

Knorpelzellen sind im Gegensatz zu anderen Zellen überdies schwer zu kultivieren. Kultivierte Knorpelzellen befinden sich in einem dedifferenzierten Zustand und ähneln morphologisch und physiologisch den Fibroblasten. Vor allem bei größeren Defekten ist jedoch die Vermehrung der autologen Chondrocyten durch Kultivierung und Passagierung für die Herstellung ausreichender Mengen an Transplantationsmaterial aus kleinen Proben notwendig.

Die Dedifferenzierung der Knorpelzellen kann bisher nur durch die Zugabe von zusätzlichen Wachstumsstimulanzien verhindert werden, was die Verwendung dieser Kulturen im in vivo Bereich erschwert, da die Wachstumsstimulanzien mit dem Immunsystem des Empfängerorganismus nachteilig wechselwirken können. Durch die bekannten Verfahren ist es des Weiteren nicht gewährleistet, dass die Zellen auch nach mehreren Passagen während der Kultivierung ihren natürlichen oder weitestgehend natürlichen Stoffwechsel aufrecht erhalten.

Die bekannten dreidimensionalen Strukturen für die Kultivierung von Knorpelzellen können in den bisherigen Verfahren zudem nicht spezifisch dem jeweiligen Knorpeldefekt angepasst werden. Es ist weiterhin nicht gewährleistet, dass die transplantierten Knorpelzellen an der Stelle des Defektes dauerhaft und differenziert verbleiben und damit die Regeneration des Knorpels ermöglichen.

Tatsächlich gibt es bisher kein Transplantationsverfahren, welches einen dauerhaften Verbleib von transplantierten Knorpelzellen an der Stelle des Defektes und eine damit verbundene Regeneration des Knorpels gewährleistet.

Das der vorliegenden Erfindung zugrunde liegende technische Problem besteht also darin, Knorpeltransplantate sowie Verfahren und Mittel zu deren Herstellung bereit zu stellen, die eine verbesserte Behandlung von Knorpelerkrankungen, insbesondere eine verbesserte Heilung beziehungsweise Regeneration des behandelten Knorpeldefektes erlauben.

Die Erfindung löst das ihr zugrunde liegende Problem durch die Bereitstellung von Mitteln zur Redifferenzierung und/oder Vermehrung von dedifferenzierten Knorpelzellen, wobei die dedifferenzierten Knorpelzellen in einer dreidimensionalen gelartigen Biomatrix kultiviert werden, dort redifferenzieren und ihre zelltypischen Stoffwechselleistungen wieder aufnehmen können.

Die erfindungsgemäße Biomatrix enthält ein aus einer, vorzugsweise frischen, Kollagenlösung neu konstituiertes Kollagengerüst einer Konzentration von mindestens 1,5 mg Kollagen/ml Biomatrix, vorzugsweise 1,5 bis 4 mg Kollagen/ml Biomatrix. Dieses Kollagengerüst wird aus einer, vorzugsweise zellfreien, sauren Kollagenlösung, vorzugsweise mit einem pH-Wert von 0,1 bis 6,9, vorzugsweise 2,0 bis 5,0, bevorzugt 3,0 bis 4,5, insbesondere 3,2 bis 4,2 und besonders bevorzugt 3,8, gewonnen, die bei 2 bis 10°C, vorzugsweise 4°C, präpariert und gelagert wurde. Diese Kollagenlösung wird zur Herstellung einer zellfreien Biomatrix bei 2 bis 10°C, vorzugsweise bei 4°C, mit einer Lösung aus Medium, insbesondere einem konventionellen Zellkulturmedium, Puffer, zum Beispiel Hepes-Puffer, und Serum, insbesondere humanem autologem Serum, versetzt und durch Erhöhung der Temperatur auf zum Beispiel Raumtemperatur oder 37°C geliert. Zur Herstellung einer zellhaltigen Biomatrix werden, vorzugsweise vorkultivierte, Zellen, zum Beispiel Knorpelzellen oder Vorläuferknorpelzellen, in die Lösung aus Medium, Puffer und Serum eingebracht und dann bei 2 bis 10°C, vorzugsweise 4°C, mit der ebenfalls 2 bis 10°C, vorzugsweise 4°C, temperierten Kollagenlösung versetzt. Die so in die Biomatrix eingebetteten Zellen können dann kultiviert und gegebenenfalls wieder herausgelöst werden. Anschließend wird bei zum Beispiel Raumtemperatur oder 37°C geliert und die Biomatrix mit Zellkulturmedium überschichtet. Das erfindungsgemäße Verfahren gestattet es vorteilhafterweise, eine Zwischenkultivierung der Zellen in einer erfindungsgemäßen Biomatrix durchzuführen, wobei die Chondrocyten, zum Beispiel der P2-Kultur, in der dreidimensionalen Biomatrix zu der erneuten Synthese von zelltypischen Matrixproteinen, insbesondere Kollagen II stimuliert werden, wohingegen in bekannten Kulturen keine Kollagenbildung nachgewiesen werden kann. Vorteilhafterweise können die Zellen anschließend wieder aus der Biomatrix herausgelöst und kultiviert werden. Durch die erfindungsgemäß ermöglichte Redifferenzierung von dedifferenzierten Knorpelzellen ist es möglich, Chondrocyten über einen längeren Zeitraum hin zu kultivieren und/oder zu vermehren, ohne dass die Zellen die spezifischen, für den Knorpelaufbau notwendigen, Syntheseleistungen verlieren. Mit Vorteil kann so auch bei einer geringen Ausgangsmenge an Knorpelgewebe genügend Zellmaterial für die Herstellung von Knorpeltransplantaten zur Verfügung gestellt werden.

Im Zusammenhang mit der vorliegenden Erfindung wird unter dem Begriff Kultivieren von Zellen ein, vorzugsweise in vitro stattfindendes, Aufrechterhalten der Lebensfunktionen von Zellen in einer geeigneten Umgebung, zum Beispiel unter Zu- und Abfuhr von Stoffwechseledukten und -produkten, verstanden, insbesondere auch eine Vermehrung der Zellen.

Im Zusammenhang mit der vorliegenden Erfindung werden unter Knorpelzellen oder Chrondrocyten natürlicherweise vorkommende oder gentechnisch veränderte Knorpelzellen oder ihre Vorläufer verstanden, die tierischer oder menschlicher Herkunft sein können.

Es ist vorgesehen, differenzierte Knorpelzellen, insbesondere unter Erhalt ihrer Differenzierung, in einer wie vorstehend charakterisierten dreidimensionalen Biomatrix zu kultivieren. Vorteilhafterweise werden die Stoffwechselleistungen der Primärkultur erhalten, ohne dass eine Dedifferenzierung der differenzierten Knorpelzellen erfolgt. Die Erfindung stellt also auch ein Kultivierungsverfahren für Chondrocyten bereit, das eine Unterdrückung der Redifferenzierung umfasst.

Es ist vorgesehen, dass in ihrer Funktion, ihrer Morphologie und/oder ihrem Differenzierungsstatus zu überprüfende Knorpelzellen in eine genannte dreidimensionale Biomatrix eingebracht, kultiviert und dabei und/oder danach überprüft werden. Vorteilhafterweise kann so beispielsweise der Stoffwechsel der Knorpelzellen in vitro untersucht werden, bevor die Knorpelzellen in vivo eingesetzt werden. Die Überprüfung kann zum Beispiel die Messung von Stoffwechselleistungen ebenso wie eine morphologische oder Funktionsüberprüfung sein.

Die Erfindung betrifft auch Screening- und Diagnoseverfahren, wobei Knorpelzellen gemäß den vorstehend beschriebenen Verfahren kultiviert und dabei oder anschließend untersucht werden können, zum Beispiel auf physiologische, morphologische und/oder molekularbiologische Parameter. Insbesondere können dabei degenerative oder traumatische Gelenkerkrankungen beziehungsweise deren Abwesenheit nachgewiesen werden. Im Rahmen dieser Verfahren können auch die Auswirkungen von potentiellen Medikamenten und/oder Krankheitserregern, Antigenen oder ähnlichem auf die kultivierten Zellen untersucht werden, zum Beispiel in drug screening Verfahren. Dabei werden in bevorzugter Ausführung die Zellen in An- und Abwesenheit des zu untersuchenden Agens kultiviert und die beobachteten Auswirkungen miteinander verglichen.

In einer vorteilhaften Ausführung werden die Knorpelzellen im Anschluss an die Kultivierung in der dreidimensionalen Biomatrix aus dieser herausgelöst, zum Beispiel mittels Kollagenasebehandlung und anschließendem Aufkonzentrieren, und in einer herkömmlichen zweidimensionalen oder dreidimensionalen Zellkultur weiter kultiviert. Vorteilhafterweise können so die Vorteile der zweidimensionalen und dreidimensionalen Kultivierung kombiniert werden.

In einer besonders vorteilhaften Ausführung werden die Knorpelzellen in eine erfindungsgemäße dreidimensionale Biomatrix eingebracht und in dieser so kultiviert, dass anschließend ein transplantierbarer Knorpelersatz, auch als Knorpeltransplantat bezeichnet, gewonnen werden kann. Dieser Knorpelersatz kann in bevorzugter Ausführung ein Gelenkknorpelersatz sein. Mit Vorteil kann so beispielsweise während der Phase des Kultivierens der Zellen bereits die Biomatrix der Form des Knorpeldefektes angepasst werden.

Eine vorteilhafte Ausführung ist ein vorgenanntes Verfahren zur Herstellung eines Knorpelersatzes, insbesondere Gelenkknorpelersatzes, wobei bevorzugt die Knorpelzellen nach Kultivierung in der dreidimensionalen Biomatrix aus dieser herausgelöst, vorzugsweise mittels Kollagenasebehandlung und Abzentrifugieren, und in -vorzugsweise- höherer Zelldichte in einer herkömmlichen zwei- oder dreidimensionalen Zellkultur weiterkultiviert werden, wobei ein transplantierbarer Knorpelersatz, insbesondere Gelenkknorpelersatz, gewonnen werden kann.

Die Erfindung betrifft auch einen Knorpelersatz, insbesondere Gelenkknorpelersatz, der nach dem erfindungsgemäßen Verfahren und einem gegebenenfalls anschließenden oder/und vorausgehenden Kultivierungsverfahren herkömmlicher Art hergestellt wurde.

Die Erfindung betrifft auch die, vorzugsweise gelartige, Biomatrix, in der die vorgenannten Kultivierungsverfahren durchgeführt werden können, und zwar als eine Biomatrix ohne Knorpelzellen als auch als eine Biomatrix mit Knorpelzellen. Im letzten Fall wird die Kombination aus Biomatrix und darin kultivierten differenzierten oder redifferenzierten Knorpelzellen auch als Zell-Matrix-Knorpel-System oder Biotransplantat bezeichnet. Dieses Biotransplantat kann direkt zur Therapie von Knorpelerkrankungen oder -defekten eingesetzt werden.

Erfindungsgemäß wird unter der Biomatrix eine Gelstruktur enthaltend Kollagen, Zellkulturmedium, Serum und Puffer, insbesondere Hepes-Puffer, verstanden. Die für die Herstellung der Biomatrix verwendete Kollagenlösung stellt eine Lösung dar, die einen hohen Anteil an nicht denaturiertem, nativen Kollagen in saurem, wässrigen Medium enthält, insbesondere mit einem pH-Wert von 0,1 bis 6,9, vorzugsweise 2,0 bis 5,0, insbesondere 3,0 bis 4,5, bevorzugt 3,2 bis 4,2 und besonders bevorzugt 3,8, zum Beispiel in Essigsäure, insbesondere 0,1 %iger Essigsäurelösung. Unter einem hohen Anteil wird ein Anteil von nicht denaturiertem Kollagen am Gesamtkollagen in Lösung von ≥ 50 %, insbesondere ≥ 60, ≥ 70, ≥ 80, ≥ 90 oder ≥ 95, insbesondere ≥ 99 % verstanden. In bevorzugter Ausführung wird kein lyophilisiertes Kollagen verwendet. Der Kollagengehalt der Lösung liegt vorteilhafterweise zwischen 3 mg Kollagen/ml Lösung bis 8 mg Kollagen/ml Lösung, vorzugsweise 6 mg Kollagen/ml Lösung. Vorteilhafterweise wird in bevorzugter Ausführungsform Kollagen verwandt, das nach Isolierung aus zum Beispiel Rattenschwänzen in 0,1 %iger Essigsäure für 3 bis 14 Tage bei 4°C unter Rühren inkubiert wurde und wobei nicht gelöste Kollagenanteile abzentrifugiert wurden. Als Zellkulturmedium wird vorteilhafterweise DMEM (Dulbecco's Modified Eagle Medium) verwendet. Es kann aber auch jedes beliebige andere Zellkulturmedium verwendet werden, welches die Kultivierung von Knorpelzellen erlaubt. Als Serum wird in bevorzugter Ausführung vorteilhafterweise autologes humanes Serum verwendet und als Puffer zum Beispiel Hepes. In bevorzugter Ausführung wird eine 3 M Konzentration von Hepes in dieser Lösung eingestellt. Der pH-Wert der Lösung aus Zellkulturmedium, Puffer und Serum beträgt in bevorzugter Ausführung 7,5 bis 8,5, zum Beispiel 7,6 bis 8,2, insbesondere 7,8. Selbstverständlich kann die Biomatrix auch weitere Faktoren, wie Wachstumsfaktoren, Adhäsionsmittel, Antibiotica, Selektionsmittel oder ähnliche, enthalten.

Die Erfindung betrifft daher auch das Verfahren zur Herstellung der Biomatrix, wobei in einem ersten Schritt frisches Kollagen, beispielsweise aus Rattenschwänzen, hergestellt wird, indem aus kollagenhaltigem Gewebe isolierte Kollagenfasern in Pufferlösung gesammelt, in Alkohol oberflächlich desinfiziert und anschließend in Pufferlösung gewaschen und anschließend in eine saure Lösung eines pH-Wertes von 0,1 bis 6,9, vorzugsweise 2,0 bis 5,0, besonders bevorzugt 3,0 bis 4,0, insbesondere 3,3, zum Beispiel eine 0,1 %ige Essigsäurelösung, überführt werden. Anschließend wird in einem weiteren Schritt das in der Lösung befindliche Kollagen bei 2 bis 10°C, insbesondere 4°C, für einige Tage, zum Beispiel 3 bis 14 Tage, gerührt, die nicht gelösten Kollagenanteile werden abzentrifugiert und eine fertige Kollagenlösung mit einem Kollagengehalt von 3 mg/ml bis 8 mg/ml bei 2 bis 10°C, zum Beispiel 4°C, aufbewahrt. Selbstverständlich ist es möglich, die Lösung in gefrorenem Zustand zu lagern, zum Beispiel bei -10°C bis -80°C, insbesondere -20°C. In einem dritten Schritt wird diese Kollagenlösung mit einer Lösung eines pH-Wertes von 7,5 bis 8,5, bevorzugt 7,6 bis 8,2, insbesondere 7,8, enthaltend doppeltkonzentriertes Zellkulturmedium, Serum und Puffer, im Verhältnis von vorzugsweise 1:1 gemischt und so eine Biomatrix erhalten, die einen pH-Wert von 7,0 bis 7,8, bevorzugt 7,4, aufweist. Zur Herstellung eines Biotransplantats wird die Lösung aus doppeltkonzentriertem Zellkulturmedium, Serum und Puffer mit vorkultivierten und abzentrifugierten Knorpelzellen gemischt, wobei vorzugsweise 2x10⁴ bis 2x10⁷ Zellen pro ml, bevorzugt 2x10⁶ Zellen, verwendet werden. Diese Lösung eines pH-Wertes von 7,5 bis 8,5, bevorzugt 7,6 bis 8,2, insbesondere 7,8 wird anschließend im Verhältnis 1:1 mit der vorgenannten Kollagenlösung bei 2 bis 10°C, insbesondere 4°C, gemischt. Anschließend wird die Gellösung in Kulturgefäße pipettiert und nach Gelieren bei 37°C mit Medium überschichtet. Sodann wird das Biotransplantat 3 bis 8 Tage kultiviert, um anschließend für Transplantationen zur Verfügung zu stehen.

Das Biotransplantat kommt in Kulturgefäßen in den Operationssaal und kann dann durch den Mediziner durch mechanische Bearbeitung, zum Beispiel mit einem Messer, in Größe, Dicke und Form dem Defekt angepasst werden. Anschließend wird das Biotransplantat, zum Beispiel mittels Gewebekleber, insbesondere Fibrinkleber, in dem Defekt fixiert. Nach circa 5 Minuten ist das Biotransplantat im Defekt fest verankert. Die Operation kann auch arthroskopisch durchgeführt werden, da das Biotransplantat flexibel ist.

Ein weiterer Einsatz des Biotransplantats stellt eine Kombination des erfindungsgemäßen Biomatrixplantats mit Knochen dar. Dabei wird das Biotransplantat zum Beispiel auf autologe Knochenzylinder aus dem Beckenkamm, zum Beispiel mittels Fibrinkleber, aufgebracht. Erfindungsgemäß wird insbesondere die Therapie von Osteochondrosis dissecans und Defekte am Hüftkopf bei cerebralparetisch bedingter Hüftluxation sowie die Sanierung von traumatischen und degenerativen Läsionen im Kniegelenk ermöglicht. Durch frühzeitige und kausale Therapie kann erfindungsgemäß mit der neuen Methode ein Fortschreiten der traumatisch oder degenerativ bedingten Gelenkerkrankungen aufgehalten werden. Für die Behandlung der Arthrose beim jüngeren Menschen steht erfindungsgemäß ein einzigartiges und neues Therapieverfahren zur Verfügung, das den endoprothetischen Gelenkersatz erheblich hinauszögert, im Einzelfall auch überflüssig macht. Auch die Therapie entzündlich bedingter Gelenkdestruktion ist als Anwendung des erfindungsgemäßen Verfahrens ermöglicht.

Die Entwicklung der erfindungsgemäßen gelartigen Biomatrix, in die autologe Zellen exakt definiert eingebettet werden können, ermöglicht erstmals die Transplantation einer definierten Menge von Knorpelzellen in einer mechanisch formbaren und belastbaren Matrix. Der erfindungsgemäße Zell-Matrix-Knorpel-System, aufgebaut aus erfindungsgemäßer Biomatrix und Knorpelzellen, kann in beliebiger Größe und Dicke hergestellt werden und durch mechanische Bearbeitung dem Defekt genau angepasst werden. Das erfindungsgemäße Biotransplantat bietet außerdem den Vorteil, dass im Gegensatz zur ACT eine zusätzliche Periostentnahme an der Tibia des Patienten entfällt. Durch die Kultivierung des erfindungsgemäßen Zell-Matrix-Knorpel-Systems in vitro ist es möglich, die Knorpelzellen vor der Transplantation hinsichtlich ihrer Matrixproduktion zu untersuchen. Dies ist eine wichtige Voraussetzung für die Herstellung von Knorpeltransplantaten, ihre Prüfung auf Funktionalität und für die Qualitätssicherung. So konnte gezeigt werden, dass die neue Synthese typischer Knorpelproteine wie zum Beispiel Kollagen Typ II, die zur Primärstabilität des Transplantats dienen, von den autologen Knorpelzellen in der Matrix in vitro gebildet werden. Erfindungsgemäß konnte durch Versuche am Kniegelenk von Minipigs gezeigt werden, dass im Vergleich zum Leerdefekt der Transplantation von Leermatrix ohne Knorpelzellen oder von Knorpelzellsuspensionen nach Peterson die erfindungsgemäßen Zell-Matrix-Knorpel-Systeme im Gelenkknorpel äußerst gut einheilen, den Defekt vollständig ausfüllen und zudem druckstabil sind.

Schließlich betrifft die Erfindung auch eine Biomatrix zur Verwendung in einem der vorgenannten Verfahren.

Die Erfindung betrifft auch Verfahren zur Behandlung von Knorpelerkrankungen oder -defekten, insbesondere degenerativer, entzündlicher und/oder traumatischer Gelenkerkrankungen, wobei ein gemäß der vorliegenden Erfindung hergestellter Knorpelersatz, Biotransplantat, und/oder gemäß der Erfindung kultivierte Knorpelzellen, in erkrankte beziehungsweise geschädigte Gewebe oder Knorpel- beziehungsweise Knochenbereiche eingesetzt, beziehungsweise gegen die erkrankten oder defekten Bereiche ausgetauscht wird beziehungsweise werden.

Die Erfindung wird anhand von Beispielen näher erläutert.

### Beispiel 1: Kollagenherstellung

Zur Herstellung einer Kollagenlösung wird kollagenhaltiges Gewebe, wie zum Beispiel Sehnen aus Rattenschwänzen, verwendet. Alle Arbeiten werden unter sterilen Bedingungen mit sterilen Materialien durchgeführt. Die Rattenschwänze werden hierfür nach Lagerung bei -20°C 5 Minuten in 70 %igem Alkohol oberflächlich desinfiziert. Anschließend wird die Haut der Rattenschwänze abgezogen und die einzelnen Kollagenfasern herausgelöst. Bei Verwendung anderer Ausgangsgewebe können gegebenenfalls vorhandene Zellen schonend durch mechanische, enzymatische oder chemische Behandlung entfernt werden. Die Kollagenfasern werden in phosphatgepufferter Salinelösung (PBS) (pH 7,2) gesammelt, in 70 %igem Alkohol für 10 Minuten oberflächlich desinfiziert und danach mit PBS gewaschen. Das Gewicht der Kollagenfasern wird bestimmt und die Fasern werden in eine 0,1 %ige Essigsäurelösung überführt (circa 8 bis 12 mg/ml). Für 3 bis 14 Tage wird dieser Ansatz bei 4°C gerührt und anschließend die nicht gelösten Kollagenteile zentrifugiert (1000 rpm, 1 Stunde, 8°C). Die fertige Kollagenlösung hat in bevorzugter Ausführung einen Kollagengehalt von 3 mg/ml bis 8 mg/ml. Das Kollagen liegt also als Ausgangsmaterial, in hohem Anteil, wie vorstehend definiert, in Lösung und nicht in Faser-, Gerüst- oder Matrixform vor. Vorzugsweise ist die erhaltene Kollagenlösung zellfrei und aus frischem, nicht lyophilisiertem Kollagen hergestellt.

### Beispiel 2: Herstellung Biomatrix beziehungsweise Biotransplantat

Alle Arbeiten werden unter sterilen Bedingungen mit sterilen Materialien durchgeführt.
- Kollagenlösung:: min. 3 mg/ml in Essigsäure
- Pufferlösung:: 77,5 ml doppeltkonzentriertes Medium (zum-Beispiel DMEM)
20 ml Serum
2,5 ml Hepes-Lösung (3 mol/l, pH 7,8)

Beide Lösungen werden bei 4°C aufbewahrt.

Die Biomatrix besteht aus einer Kollagenlösung in 0,1 %iger Essigsäure (Kollagen aus Rattenschwanz mit einem Kollagengehalt von 3 mg/ml bis 8 mg/ml, bevorzugt 6 mg/ml) und einer Pufferlösung aus doppeltkonzentriertem Medium, Serum und Hepes-Lösung. Kurz nach Mischen dieser beiden Komponenten im Verhältnis von vorzugsweise 1:1 geliert, bei Temperaturen oberhalb von 4°C, zum Beispiel Raumtemperatur, eine dreidimensionale neu konstituierte Kollagenstruktur aus.

Zur Herstellung des Biotransplantats, also einer zellhaltigen Biomatrix, werden in üblicher Weise vorkultivierte und abzentrifugierte. (1000 rpm, 10 min., RT) 2x10⁴ bis 2x10⁷ Knorpelzellen/ml in der Pufferlösung, bevorzugt 2x10⁶, aufgenommen, suspendiert und mit gleichen Teilen der Kollagenlösung bei 4°C gemischt. Diese Gellösung wird in Kulturgefäße pipettiert und nach Gelieren bei 37°C zu einer neu konstituierten Kollagenstruktur mit darin eingebetteten Knorpelzellen mit Medium überschichtet. Das Material steht dann nach 3 bis 8 Tagen Kulturdauer für die Transplantation zur Verfügung.

Sofern gewünscht, können die Zellen durch Kollagenasebehandlung und anschließendes Abzentrifugieren aus der Biomatrix wieder freigesetzt und gewonnen werden. Die erhaltenen differenzierten Zellen können Ausgangspunkt weiterer Kultivierungen sein.

### Beispiel 3: Transplantation am offenen Gelenk und arthroskopische Applikation

Sowohl bei der Transplantation am offenen Gelenk als auch bei der arthroskopischen Applikation wird der Zell-Matrix Knorpel zunächst in seiner Größe und Form dem Knorpeldefekt mechanisch angepasst. Anschließend wird das Transplantat in den Defekt eingebracht und dort mittels Fibrinklebers fixiert. Da es sich bei dem beschriebenen Zell-Matrix Knorpel-System um einerseits flexibles, aber dennoch formstabiles Material handelt, ist es möglich, dieses Biotransplantat in den röhrenförmigen Arthroskopieinstrumenten quasi einzurollen, und sie so in den Defekt einzubringen. Durch ihre Formstabilität nehmen die Transplantate am Bestimmungsort wieder ihre ursprüngliche Form an und können nun mit Fibrinkleber im Defekt befestigt werden.

### Beispiel 4: Ergebnisse von PCR-Untersuchungen

In der folgenden Tabelle spiegeln sich die Ergebnisse von PCR-Untersuchungen wieder. Ziel dieser Untersuchungen war es, aufzuzeigen, dass die erfindungsgemäße Kultivierung in einer erfindungsgemäßen gemäß Beispiel 1 und 2 hergestellten Biomatrix zu einer Redifferenzierung von Chondrocyten führt. Aus der Tabelle wird ersichtlich, dass ausgehend von einer PO-Kultur im Verlaufe der darauffolgenden konventionell durchgeführten Passagen P1 und P2 die Fähigkeit der Chondrocyten verloren geht, Kollagentyp II herzustellen. Die Zellen dedifferenzieren im Verlaufe dieser Passage. Eine P2-Passage in einer erfindungsgemäßen Biomatrix führt dagegen zur Redifferenzierung dedifferenzierter Chondrocyten, was an der wiedergewonnenen Fähigkeit, Kollagentyp II herzustellen, belegt wird.

**Tabelle: Ergebnisse der PCR-Untersuchungen: Aus den Chondrocyten der einzelnen Kulturen wurde die RNA isoliert und in cDNA umgeschrieben.**

| | PO-2D | P1-2D | P2-2D | P2-Kollagen 2x10⁶/ml |
|---|---|---|---|---|
| | 3 Wochen | 2 Wochen | 3 Wochen | 3 Wochen |
| β2Microglobulin | + | + | + | + |
| Kollagen Typ I | + | + | + | + |
| Kollagen Typ II | + | - | - | + |
| Kollagen Typ XI | + | + | + | + |
| Bmp 2 | + | + | + | + |
| Bmp 4 | + | + | + | + |
| Bmp 7 | - | - | - | - |

Die Expression (Transcription) chondrocytenspezifischer Gene wurde durch PCR-Amplifikation mit geeigneten Primern und anschließender Gelelektrophorese getestet:
- β2-Mikroglobulin ist ein konstitutiv exprimiertes Gen und dient als Positivkontrolle;
- Kollagen Typ II und XI sind knorpelspezifische Kollagen-Typen;
- Bone morphogenetic proteins (Bmp) 2,4 und 7 spielen eine Rolle bei der Differenzierung des Knorpelgewebes.

## Patentansprüche

1. Verfahren zur Herstellung von Kollagenbiomatrix mit den Schritten:
Präparieren von Kollagenfasern aus Rattenschwänzen;
Überführen der Kollagenfasern in eine essigsaure Lösung mit einem pH-Wert von 3,0 bis 4,0 ;
Inkubieren der Kollagenfasern in der essigsauren Lösung bei einer Temperatur von 2 bis 10 °C und für einen Zeitraum von 3 bis 14 Tagen;
Abtrennen der nicht gelösten Kollagenteile, sodass eine Kollagenlösung mit einem Kollagengehalt von 3 bis 8 mg/ml erhalten wird; und
Mischen der erhaltenen Kollagenlösung mit einer Pufferlösung eines pH-Wertes von 7,5 bis 8,5 bei einer Temperatur von 2°C bis 10°C und Erhöhen der Temperatur zum Gelieren, sodass eine Biomatrix mit von 1,5 bis 4,0 mg Kollagen pro 1 ml Biomatrix und mit einem pH-Wert von 7,0 bis 7,8 erhalten wird.

2. Verfahren nach Anspruch 1, wobei die Kollagenfasern in der essigsauren Lösung in einem Anteil von 8 bis 12 mg Kollagenfasern pro 1 ml der essigsauren Lösung vorliegen.

3. Verfahren nach Anspruch 1 oder 2, wobei die essigsaure Lösung eine 0,1 %ige Essigsäurelösung ist.

4. Kollagenlösung, hergestellt durch das Verfahren nach einem der Ansprüche 1 bis 3.

5. Biomatrix, hergestellt durch das Verfahren nach einem der Ansprüche 1 bis 4.

## Claims

1. Method for preparing a collagen biomatrix, comprising the steps of:
dissecting of collagen fibres from rat tails;
transferring said collagen fibres into an acetic acid solution having a pH of 3.0 to 4.0;
incubating said collagen fibres in said acetic acid solution for 3 to 14 days at a temperature of 2 to 10°C;
separating non-dissolved collagen parts such that a collagen solution having a collagen content of 3 mg/ml to 8 mg/ml is obtained; and
mixing the obtained collagen solution with a buffer solution having a pH of 7.5 to 8.5 at a temperature of 2°C to 10°C and elevating the temperature for gelling such that a biomatrix with a collagen content of 1.5 to 4.0 mg per 1 ml biomatrix having a pH of 7.0 to 7.8 is obtained.

2. Method according to claim 1, wherein the collagen fibres are present in the acetic acid solution in a proportion of 8 to 12 mg of collagen fibres per 1 ml acetic acid solution.

3. Method according to claim 1 or 2, wherein the acetic acid solution is a 0.1 % acetic acid solution.

4. Collagen solution as prepared by means of the method according to one of the claims 1 to 3.

5. Biomatrix as prepared by means of the method according to one of the claims 1 to 4.

## Revendications

1. Procédé concernant la fabrication d'une biomatrice au collagène comprenant les étapes de:
Préparation de fibres de collagène à partir de queues de rat ;
Disposition des fibres de collagène dans une solution d'acide acétique dont le taux de pH est compris entre 3,0 et 4,0 ;
Incubation des fibres de collagène dans la solution d'acide acétique à une température comprise entre 2 et 10°C et ce pendant une durée de 3 à 14 jours ;
Séparation des parties de collagène non dissoutes, en vue d'obtenir une solution au collagène dont la teneur en collagène sera comprise entre 3 et 8 mg/ml ; et
Mélange de la solution au collagène ainsi obtenue avec une solution tampon dont le taux de pH est compris entre 7,5 et 8,5 à une température qui se situe entre 2°C et 10°C et augmentation de la température jusqu'à gélification en vue d'obtenir une biomatrice avec une teneur en collagène comprise entre 1,5 et 4,0 mg par biomatrice de 1 ml et un taux de pH compris entre 7,0 et 7,8.

2. Procédé selon la revendication 1, dans lequel la proportion des fibres de collagène qui se trouvent dans la solution d'acide acétique se situe entre 8 et 12 mg pour 1 ml de solution d'acide acétique.

3. Procédé selon la revendication 1 ou 2, dans lequel la solution d'acide acétique est une solution d'acide acétique à 0,1%.

4. Solution de collagène fabriquée au moyen du procédé selon une des revendications 1 à 3.

5. Biomatrice fabriquée au moyen du procédé selon une des revendications 1 à 4.
